# EUROPEAN PATENT SPECIFICATION

(11) **EP 0 531 425 B1**
(45) Date of publication and mention of the grant of the patent: **14.08.2002**
(21) Application number: 91910904.1
(22) Date of filing: 30.05.1991
(51) Int. Cl.: A61K 38/18, A61P 19/00

(54) **Combination of IGF-I and TGF-beta for bone regeneration**
Kombination aus IGF-I und TGF-beta zur Knochenregenerierung
Combinasion de IGF-I et de TGF-beta pour la régeneration osseusse

(30) Priority: 30.05.1990 US 530649
(43) Date of publication of application: 17.03.1993
(73) Proprietor: THE PRESIDENT AND FELLOWS OF HARVARD COLLEGE, Cambridge, MA 02114 (US); INSTITUTE OF MOLECULAR BIOLOGY INC., Boston MA 02115 (US)
(72) Inventor: ANTONIODES, Harry, N., Newton, MA 02158 (US); LYNCH, Samuel, E., Beverly, MA 01915 (US)
(74) Representative: Deans, Michael John Percy
(86) International application number: US9103829
(87) International publication number: WO9118622

(56) References cited:
- EP-A- 0 264 074
- EP-A- 0 267 015
- EP-A- 0 280 460
- EP-A- 0 312 208
- EP-A- 0 323 842
- EP-A- 0 436 469
- WO-A-88/03409
- WO-A-88/05787
- WO-A-89/11293
- WO-A-90/00060
- WO-A-90/00569
- US-A- 4 738 921
- US-A- 4 816 561
- US-A- 4 885 163
- BONE, vol. 10, no. 2, 1989, pages 131-138, XP002033922 J.E. PICH ET AL.: "Study of the Growth Factor Requirements of Human Bone-derived Cells: A Comparison with Human Fibroblasts"
- REV. MED. BRUX., vol. 10, no. 10, December 1989, pages 419-423, XP002033923 P. BERGMANN: "Contrôle local du remaniement osseux"
- ENDOCRINOL. METAB. CLIN. NORTH. AM., vol. 18, no. 4, December 1989, pages 903-918, XP002033924 E. CANALIS ET AL.: "The Role of Growth Factors in Skeletal Remodeling"
- J. DENTAL. RES., vol. 68, no. spec, 1989, page 394 XP002033925 S.E. LYNCH ET AL.: "Effect of Insulin-like Growth Factor on Periodontal Regeneration"
- DTSCH. MED. WOCHENSCHR., vol. 115, no. 50, 14 December 1990, pages 1921-1926, XP002033926 K.G. KUKOSCHKE ET AL.: "Bedeutung von Protein-Wachstumfaktoren für die lokale Regulation des Knochenwachstums"
- INT. CONGR. SER.-EXCERPTA MED., NO. GROWTH FACTORS HEALTH DIS., vol. 925, 1990, pages 89-101, XP002033927 A.B. ROBERTS ET AL.: "Transforming Growth Factor-Beta ..."
- J. CLIN. PERIODONTOL., vol. 16, no. 8, September 1989, pages 545-548, XP002033928 S.E. LYNCH ET AL.: "A combination of platelet-derived and insulin-like growth factors enhances periodontal regeneration"
- ANN. SURG., vol. 211, no. 3, March 1990, pages 288-294, XP000673223 G.A. KSANDER ET AL.: "Exogenous Transforming Growth Factor-Beta 2 Enhances Connective Tissue Formation and Wound Strength in Guinea Pig Dermal Wounds Healing by Secondary Intent"
- FASEB J., vol. 2, no. 5, 1988, page abstract 4924 XP002033929 C.J. SHAAR ET AL.: "The Anabolic Effects of Recombinant DNA-Dericed Human Insulin-like Growth Factor-II (LY255986) in Immature Hypophysectomized Rats"
- FASEB J., vol. 3, no. 3, 1989, page A282 XP002033930 C.J. SHAAR ET AL.: "The Effects of Topically-Applied Recombinant Human Insulin-like Growth Factor-II (LY255986) on the Wound Healing Process in Diabetic Rats"
- SCIENCE, vol. 237, no. 4820, 11 September 1987, pages 1333-1336, XP000673224 T.A. MUSTOE ET AL.: "Accelerated Healing of Incisional Wounds in Rats Induced by Transforming Growth Factor-beta"
- DIALOG INFORMATION SERVICES, FILE 155: MEDLINE (R), ACCESSION NUMBER 05997625, & MEAD JOHNSON SYMP PERINAT DEV MED, no. 32, 1988, pages 12-20, XP002033931 M.M. RECHLER: "Insulin-like Growth Factors: Polypeptides with Diverse Physiological Roles"
- DATABASE WPI Week 8705 Derwent Publications Ltd., London, GB; AN 87-034010 XP002033932 & JP 61 291 521 A (US GOVERNMENT) , 22 December 1986
- J. INVEST. DERMATOL., vol. 94, no. 6, June 1990, pages 777-780, XP000673221 N.W. DELAPP ET AL.: "Effect of Basic Fibroblast Growth Factor (bFGF) and Insulin-Like Growth Factors Type I (IGF-I) and Type II (IGF-II) on Adult Human Keratinocyte Growth and Fibronectin Secretion"
- SCI. TECH. PRAT. PHARM., vol. 5, no. 8-9, 1989, pages 567-575, XP000673218 D. BARRITAULT: "Les facteurs de croissance cellulaire; Perspectives d'application en biologie et en pharmacologie"
- ANN. N.Y. ACAD. SCI, vol. 593, 1990, pages 124-134, XP000673222 A.J. AMMANN ET AL.: "Transforming Growth Factor-beta; Effect on Soft Tissue Repair"
- Science, Vol. 237, issued 11 September 1987, MUSTOE et al., "Accelerated Healing of Incisional Wounds in Rats Induced by Transforming Growth Factor-B", pages 1333-1336, see entire document.

## Description

This invention relates to the use of certain growth factors for the manufacture of a medicament for regenerating bone in a mammal.

Growth factors are polypeptide hormones which stimulate a defined population of target cells. Examples of growth factors include platelet-derived growth factor (PDGF), insulin-like growth factor (IGF-I), transforming growth factor beta (TGF-β), transforming growth factor alpha (TGF-a), epidermal growth factor (EGF), and fibroblast growth factor (FGF).

TGF-β is a multifunctional regulatory polypeptide synthesized by many cell types and sequestered in human platelets in amounts similar to PDGF. The in vitro biological effects of TGF-β are dependent upon the presence of other growth factors: TGF-β in the presence of PDGF stimulates fibroblast growth, and in the presence of EGF inhibits fibroblasts (Roberts, et al. Proc. Natl. Acad. Sci, USA 82:119). TGF-β inhibits proliferation of epithelial cells in vitro (Shipley et al., 1986, Cancer Res. 46:2068), and in vivo stimulates DNA, total protein, and collegen synthesis when injected into wound chambers (Sporn et al., 1986, Science 219:1329). The breaking strength of incisional wounds increases in a dose dependent manner after application of TGF-β (Mustoe, et al., Science 237:1333).

IGF-I is synthesized de novo in the liver and secreted into the plasma. In vitro, IGF-I can promote DNA synthesis in both mesenchymal and epithelial cells (Van Wyk 1984, Hormonal Proteins and Peptides, Li, ed.). Addition of IGF-I in vivo by itself does not promote wound healing, but when added with PDGF the combination stimulates connective tissue and epithelial and bone cell proliferation (Lynch, et al., 1987, Proc. Natl. Acad. Sci., USA 84:7696, Lynch et al., 1987, J. Clin. Periodontol. 16:545).

WO 89/11293 discloses the use of a combination of purified TGF-β and purified IGF-I for healing external wounds in a mammal. Compositions comprising TGF-β and IGF-I are also disclosed.

Piché, J.E. et al in Bone, 10, 131-138 (1989) reported the results of a study of the growth factor requirements of human bone-derived cells in comparison with human fibroblasts. In this study, it was found that bone-derived cells proliferated in basal medium supplemented with platelet-poor plasma. The rate of proliferation was enhanced by additional supplementation with PDGF and further increased when a combination of growth factors was added (PDGF, TGF-β and EGF). In contrast, fibroblasts did not proliferate in basal medium supplemented with platelet-poor plasma and, although the addition of PDGF alone stimulated fibroblast proliferation, supplementation with other growth factors did not further enhance the response of fibroblasts to PDGF. These results emphasise the differences in proliferative responses between human bone-derived cells and human fibroblasts, and indicate that the factors responsible for osseous regeneration in vivo may differ from those factors which regulate repair of soft tissue wounds.

Bergmann, P. in Rev.Méd.Brux.,10(10),419-423(1989) reveals that several growth factors are secreted by cells of the osteoblastic lineage and by other bone cells and are stored in the bone matrix from which they are released and activated during resorption. The most important of these are said to be IGF-I, IGF-II and TGF-β which stimulate replication of the osteoblastic precursors and collagen synthesis by osteoblasts, and could play a role in the coupling between resorption and formation. It is also noted that TGF-β seems to inhibit replication of osteoclast precursors and could thus stop the resorption process.

In one aspect, the invention features concomitant use of purified Insulin-like growth factor -I (IGF-I) and purified transforming growth factor beta (TGF-β) for the manufacture of a medicament for regenerating bone in a mammal, e.g., a human patient. In a second aspect, the invention provides use of a composition comprising IGF-I and TGF-β for the manufacture of a medicament for regenerating bone in a mammal.Regeneration is achieved by administering to the patient, preferably by application to the area of injured or depleted bone, an effective amount of a composition that includes purified Insulin-like growth factor-I and purified transforming growth factor beta. The composition aids in regeneration, at least in part, by promoting the growth of bone cells and bone matrix. Bone regeneration using the composition of the invention is more effective than that achieved in the absence of treatment (i.e., without applying exogenous agents) or by treatment with purified Insulin-like growth factor-I or purified transforming growth factor beta alone.

In preferred embodiments of this aspect of the invention, the composition is prepared by combining purified IGF-I and TGF-β. Most preferably, purified IGF-I and TGF-β are combined in a weight-to-weight ratio of between 25:1 and 1:25, preferably between 2:1 and 1:2, and more preferably 2:1 or 1:1.

The term "purified" as used herein refers to IGF-I or TGF-β which, prior to mixing with the other component, is 95% or greater, by weight, IGF-I or TGF-β, i.e., is substantially free of other proteins, lipids, and carbohydrates with which it is naturally associated.

A purified protein preparation will generally yield a single major band on a polyacrylamide gel for each IGF-I' or TGF-β component. Most preferably, the purified IGF-I' or TGF-β used in the composition is pure as judged by amino-terminal amino acid sequence analysis.

IGF-I and TGF-β are all commercially available and may be obtained using recombinant DNA technology or by solid phase peptide synthesis. The purified TGF-β may be obtained from human platelets or by recombinant DNA technology. Thus, by the terms "IGF-I", "TGF-β", we mean both platelet-derived and recombinant materials of mammalian, preferably primate, origin; most preferably, the primate is a human, but can also be a chimpanzee or other primate. Recombinant TGF-β can be recombinant monomer or homodimer, made by inserting into cultured prokaryotic or eukaryotic cells a DNA sequence encoding a subunit, and then allowing the translated subunits to be processed by the cells to form a homodimer.

The compositions described above provide a fast effective method for regenerating bone, e.g., injured, infected, or malignant bone. The compositions promote a significant increase in new bone formation, especially adjacent root and periosteal surfaces.

Other features and advantages of the invention will be apparent from the following description of the preferred embodiments thereof, and from the claims.

We now describe preferred embodiments of the invention.

Bone wounds following injury, infection, or malignancy, are treated with IGF-I/TGF-β. Recombinant human IGF-I is commercially available from Amgen Biologicals (Thousand Oaks, CA.). Purified human and porcine TGF-β are commercially available from R&D Systems, Inc. (Minnesota, MN).

TGF-β was purified from human or porcine platelets by the method of Assoian (1983, J. Biol. Chem. 258: 7155). Briefly, platelet-rich plasma (20-30 units, 2-5 days old) was centrifuged (3200xg 30 min, at 4°C) to remove plasma proteins. The platelets were then washed twice in 500ml portions of Tris-HCl/citrate buffer, and recentrifuged. The washed platelets were then added to a solution of acid ethanol and then immediately extracted in a homogenizer. After incubation overnight at 4°C, precipitated proteins were removed by centrifugation and the supernatant adjusted to pH 3 using NH₄OH. TGF-β was precipitated by addition of ethanol (2 volumes at 0°C) and ethyl ether (4 volumes at 0°C). The precipitate was collected by centrifugation and suspended in 1M acetic acid (10ml). The supernatant was separated from the precipitate by centrifugation and placed on a Bio-Gel P60 gel filtration column (4.4 x 115 cm), with a flow rate of 20ml/hr, equilibrated in 1M acetic acid. Five milliliter fractions were collected and assayed for biological activity using growth inhibition of BALB/MK cells and anchorage-independent growth of non-neoplastic NRK fibroblasts. Fractions containing peak activity were pooled, lyophilized, and redissolved in 0.5ml of 1M acetic acid containing 8M ultra-pure urea (Schwartz/Mann) and gel filtered at a flow rate of 3ml/hr on a Bio-Gel P60 column (1.6 x 85cm). Aliquots of column fractions were tested for TGF-β activity as described above. Fractions containing peak TGF-β activity were pooled, dialized against 1M acetic acid to remove urea, and added to a C-18 (synchropak) HPLC column in 0.1% triflouroacetic acid and eluted with a 20-50% acetonitrile gradient. Biologically active fractions were pooled, and final purity checked by SDS-PAGE and amino acid analysis for known properties of TGF-β.

Recombinant TGF-β can be prepared by standard techniques. For example, oligonucleotide probes designed on the basis of the protein sequence of TGF-β can be used for the isolation of TGF-β exons in a human genomic DNA or a cDNA library, using the technique described in Birynch (1985, Nature 316: 701). The gene for TGF-β is isolated, cloned into standard expression vectors, and transfected into mammalian cells, from which TGF-β is then purified using standard methods.

To determine the effectiveness of TGF-β/IGF-I preparations in promoting periodontium and/or bone growth, the following experiments may be performed.

Beagle dogs with naturally occurring periodontal disease are selected on the basis of an initial radiographic examination. The teeth which exhibit 30% to 80% bone loss are initially scaled using ultrasonic instruments. Surgical flaps and root planing techniques are then performed, and the experimental teeth are treated with a composition containing purified TGF-β and IGF-I in a pharmaceutically acceptable carrier substance, e.g., commercially available inert gels, e.g., methyl cellulose. Teeth in the remaining quadrants receive control gel alone, or pure TGF-β or IGF-I alone. Block biopsies of the teeth and bone are taken periodically following surgery and prepared for histologic evaluation using standard demineralizing and processing techniques. Histologic analysis of periodontal and bone specimens will indicate whether, adjacent to the root surfaces of experimental specimens (i.e., those treated with the TGF-β/IGF-I combination), distinct areas of new bone formation are present and whether a deposit resembling cementum is present on the root surface adjacent to the new bone. New bone may also be present on the periosteal surface of the specimens. In addition, abundant proliferation of osteoblast-like cells may be present adjacent to the newly formed bone and newly formed collagen fibers may insert into the newly formed cementum.

In contrast, the control specimens may appear as follows: there will be little evidence of new bone formation, an absence of new cementum-like deposits, and connective tissue may be oriented perpendicular to the bony surface appearing to form a "cap" over the original bone.

To determine the optimal ratio of IGF-I to TGF-β, combinations in which the weight to weight ratio of IGF-I to TGF-β range from 25:1 to 1:25 can be evaluated as described above for bone regeneration experiments.

Other embodiments are within the following claims. For example, IGF-I and TGF-β can be obtained by standard recombinant DNA technology using nucleic acid having a base sequence identical to that of the naturally occurring gene encoding IGF-I' or TGF-β in a human or other mammal. Further, this nucleic acid may be modified by conservative base substitutions such that it encodes the same amino acid sequence of naturally occurring IGF-I or TGF-β; or modified with base substitutions which encode a different amino acid sequence to that naturally occurring, but the protein product of which has substantially the same properties as the naturally occurring proteins.

## Claims

1. Concomitant use of purified Insulin-like growth factor-I (hereafter "IGF-I") and purified transforming growth factor beta (hereafter "TGF-β") for the manufacture of a medicament for regenerating bone in a mammal.

2. Use of a composition comprising IGF-I and TGF-β for the manufacture of a medicament for regenerating bone in a mammal.

3. Use according to claim 2 in which the composition comprises the specified growth factors together with a pharmaceutically acceptable carrier.

4. Use according to any one of Claims 1 to 3, wherein the weight to weight ratio of IGF-I to TGF-β is between 1:25 and 25:1.

5. Use according to Claim 4, wherein said ratio is between 1:4 and 25:1.

6. Use according to Claim 4, wherein said ratio is between 1:2 and 10:1.

7. Use according to Claim 4 or Claim 6 wherein said ratio is between 1:2 and 2:1.

## Patentansprüche

1. Begleitende Verwendung von gereinigtem insulinartigem Wachstumsfaktor I (nachfolgend "IGF-I" genannt) und gereinigtem transformierendem Wachstumsfaktor Beta (nachfolgend "TGF-β" genannt) zur Herstellung eines Medikaments zur Knochenregeneration in einem Säugetier.

2. Verwendung einer IGF-I und TGF-β umfassenden Zusammensetzung zur Herstellung eines Medikaments zur Knochenregeneration in einem Säugetier.

3. Verwendung nach Anspruch 2, wobei die Zusammensetzung die vorgegebenen Wachstumsfaktoren zusammen mit einem pharmazeutisch akzeptablen Träger umfasst.

4. Verwendung nach einem der Ansprüche 1 bis 3, wobei das Gewichtsverhältnis zwischen IGF-I und TGF-β zwischen 1:25 und 25:1 liegt.

5. verwendung nach Anspruch 4, wobei das genannte Verhältnis zwischen 1:4 und 25:1 liegt.

6. Verwendung nach Anspruch 4, wobei das genannte Verhältnis zwischen 1:2 und 10:1 liegt.

7. Verwendung nach Anspruch 4 oder Anspruch 6, wobei das genannte Verhältnis zwischen 1:2 und 2:1 liegt.

## Revendications

1. Utilisation concomitante d'un facteur-I de croissance insulinoïde purifié (désigné ci-après par « IGF-I ») et d'un facteur bêta de croissance transformant purifié (désigné ci-après par « TGF-β ») pour la fabrication d'un médicament pour régénérer de l'os chez un mammifère.

2. Utilisation d'une composition comprenant IGF-I et TGF-β pour la fabrication d'un médicament pour régénérer de l'os chez un mammifère.

3. Utilisation selon la revendication 2, dans laquelle la composition comprend les facteurs de croissance spécifiés ainsi qu'un véhicule pharmaceutiquement acceptable.

4. Utilisation selon l'une quelconque des revendications 1 à 3, dans laquelle le rapport pondéral de l'IGF-I au TGF-β est compris entre 1:25 et 25:1.

5. Utilisation selon la revendication 4, dans laquelle ledit rapport est compris entre 1:4 et 25:1.

6. Utilisation selon la revendication 4, dans laquelle ledit rapport est compris entre 1:2 et 10:1.

7. Utilisation selon la revendication 4 ou la revendication 6, dans laquelle ledit rapport est compris entre 1:2 et 2:1.
